# EUROPÄISCHE PATENTANMELDUNG

(11) **EP 1 348 395 A1**
(43) Veröffentlichungstag der Anmeldung: **01.10.2003**
(21) Anmeldenummer: 03006997.5
(22) Anmeldetag: 27.03.2003
(51) Int. Cl.: A61B 19/02

(54) **Abfallbehälter mit Verschlusslasche**

(30) Priorität: 28.03.2002 DE 10214163
(71) Anmelder: Udo Heisig GmbH, 85640 Putzbrunn (DE)
(72) Erfinder: Heisig, Udo, 85640 Putzbrunn (DE)
(74) Vertreter: Feldkamp, Rainer, Dipl.-Ing.

(57) **Zusammenfassung**

Ein Abfallbehälter, insbesondere für medizinische Abfälle, weist eine Einfüllöffnung (1) auf, die durch einen Verschlußdeckel (2) verschließbar ist, der mit dem Abfallbehälter über eine Gelenkverbindung (3) gelenkig verbunden ist, wobei zur Arretierung des Verschlußdeckels (2) nach dem Befüllen des Abfallbehälters bezüglich der Einfüllöffnung (1) Rasteinrichtungen (4, 7) vorgesehen sind. Der Verschlußdeckel (2) weist an einer der Gelenkverbindung (3) gegenüberliegenden Position eine sich nach außen hin erstreckende Lasche (4) auf, die gelenkig mit dem Verschlußdeckel (2) verbunden ist und einen ersten Teil der Rasteinrichtungen bildet. Der Abfallbehälter weist eine Verschlußhülse (7) auf, in die die Lasche (4) einsteckbar ist und die einen zweiten Teil der Rasteinrichtungen bildet. Auf der Außenseite der Lasche (4) und der Innenseite der Verschlußhülse (7) sind jeweils zusammenwirkende Rastverzahnungen (4a, 7a) angeordnet, die einen unlösbaren Rasteingriff zwischen der Lasche (4) und der Verschlußhülse (7) beim Einstecken der Lasche (4) in die Verschlußhülse (7) ergeben.

## Beschreibung

Die vorliegende Erfindung betrifft einen Abfallbehälter der im Oberbegriff des Patentanspruchs 1 genannten Art.

Derartige Abfallbehälter, die beispielsweise aus der EP 0 343 680 B1 bekannt sind, werden insbesondere im medizinischen Bereich verwendet, um medizinische Abfälle, insbesondere spitzes oder scharfkantiges Abfallgut wie Injektionsspritzen, Kanülen, Skalpelle und Glasbruch, aber auch gebrauchtes Verbandsmaterial oder sonstige Abfälle zu entsorgen, die beispielsweise durch Viren oder Bakterien kontaminiert sein können.

Bei derartigen Abfallbehältern ist es erforderlich, daß der Einfüllöffnungs-Verschlußdeckel vor der Entsorgung des Abfallbehälters endgültig derart verschlossen wird, daß er zumindest ohne Gewaltanwendung nicht mehr geöffnet werden kann. Diese Anforderung ergibt sich daraus, daß ein Verletzungs- oder sogar Infektionsrisiko durch in dem Abfallbehälter enthaltene kontaminierte Abfälle für die Personen vermieden werden soll, die vor oder während der Entsorgung mit dem Abfallbehälter hantieren. Der Verschlußdeckel kann entweder die gesamte Öffnung des Abfallbehälterkörpers verschließen, oder diese Öffnung kann zunächst durch einen Abfallbehälterdeckel verschlossen sein, in dem dann eine Einfüllöffnung ausgebildet ist, die durch den Verschlußdeckel verschließbar ist.

Der vorliegenden Erfindung liegt die Aufgabe zugrunde, einen Abfallbehälter der eingangs genannten Art dahingehend weitertzubilden, daß der Verschlußdeckel eine erste lösbare Schließstellung und eine zweite Schließstellung aufweist, in der ein erneutes Öffnen des Verschlußdeckels weitgehend unmöglich gemacht wird.

Diese Aufgabe wird durch die im kennzeichnenden Teil des Patentanspruchs 1 angegebenen Merkmale gelöst.

Vorteilhafte Weiterbildungen und Ausgestaltungen der Erfindung ergeben sich aus den Unteransprüchen.

Der erfindungsgemäße Abfallbehälter ermöglicht ein Verschließen der Einfüllöffnung durch einen Verschlußdeckel, der über eine Gelenkverbindung gelenkig mit dem Abfallbehälter selbst oder mit einem diesen verschließenden Abfallbehälterdeckel verbunden ist, wobei im letztgenannten Fall die durch den Verschlußdeckel zu verschließende Einfüllöffnung in dem Abfallbehälterdeckel angeordnet ist. Der Verschlußdeckel kann in einer ersten Schließstellung die Einfüllöffnung lösbar verschließen, wobei die Lasche von dem Verschlußdeckel aus nach außen vorspringt und ein nachfolgendes Öffnen des Verschlußdeckels aus der ersten Verschlußstellung heraus erleichtert.

Andererseits ist diese Lasche gelenkig mit dem Verschlußdeckel verbunden und kann gegenüber diesem so verschwenkt werden, daß sie in eine Verschlußhülse einssteckbar ist, die am Außenumfang des Abfallbehälters oder des Abfallbehälterdeckels angeordnet ist, wobei die Verschlußhülse und zumindest der in diese einsteckbare Teil der Lasche zusammenwirkende Rastverzahnungen aufweisen, die ein erneutes Öffnen des Verschlußdeckels verhindern, wobei ein allenfalls mögliches gewaltsames Öffnen des Verschlußdeckels sofort erkennbar ist.

Die Verschlußhülse umgibt in der endgültigen Schließstellung des Verschlußdeckels den in sie eingesteckten Teil der Lasche, wobei die Rastverbindung vorzugsweise unzugänglich im Inneren der Verschlußhülse liegt.

Wenn ein Abfallbehälterdeckel verwendet wird, so kann dieser in bekannter Weise ebenfalls unlösbar und flüssigkeitsdicht mit dem Behälterkörper über Rasteinrichtungen verbunden werden.

Ausführungsbeispiele der Erfindung werden im folgenden anhand der Zeichnungen noch näher erläutert.

In der Zeichnung zeigen:
Figur 1 eine perspektivische Ansicht einer ersten Ausführungsform eines Abfallbehälterdeckels mit gelenkig daran befestigtem Verschlußdeckel, wobei der Abfallbehälterkörper nicht dargestellt ist;
Figur 2 eine Draufsicht auf die in Figur 1 gezeigte Ausführungsform des Abfallbehälterdeckels und des Verschlußdeckels;
Figur 3 eine Schnittansicht entlang der Linie 3-3 nach Figur 2;
Figur 4 eine weitere Ausführungsform eines Abfallbehälterdeckels mit einem Verschlußdeckel in geöffnetem Zustand;
Figur 5 eine der Figur 4 entsprechende Darstellung des Abfallbehälterdeckels, wobei sich der Verschlußdeckel in der ersten (lösbaren) Verschlußstellung befindet;
Figur 6 eine vereinfachte Schnittansicht im wesentlichen entlang der Linie VI-VI nach Figur 2, die ausführliche Einzelheiten der Rasteinrichtungen zum Verschließen des Verschlußdeckels gegenüber dem Abfallbehälterdeckel zeigt, wobei der obere Rand des Abfallbehälterkörpers nur schematisch angedeutet ist.

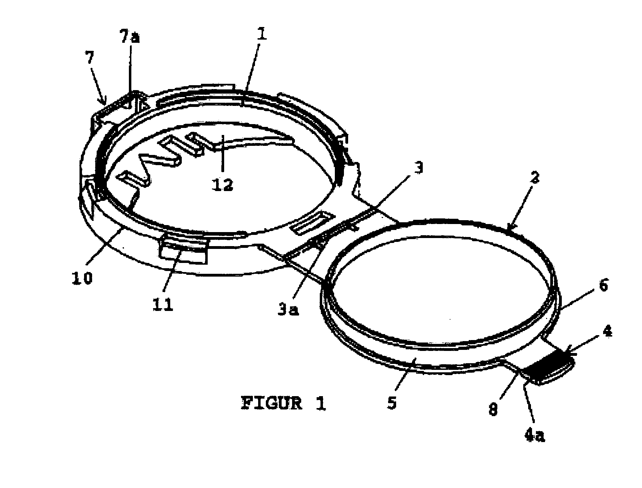

In Figur 1 ist eine perspektivische Ansicht einer ersten Ausführungsform eines Abfallbehälterdeckels 10 gezeigt, der beispielsweise mit einem Abfallbehälterkörper gemäß der EP 0 343 680 verwendet werden kann, der aus Vereinfachungsgründen hier nicht näher dargestellt ist.

Mit dem Abfallbehälterdeckel 10 ist über ein Filmscharnier 3 ein Verschlußdeckel 2 gelenkig verbunden. Der Abfallbehälterdeckel 10 weist eine Einfüllöffnung 1 auf, in der ein Steg 12 angeordnet ist, der Kerben beispielsweise zum Abstreifen von Spritzen und dergleichen aufweist.
Der Verschlußdeckel 2 weist einen ringförmigen Flansch 5 auf, dessen Durchmesser an den Innendurchmesser der Einfüllöffnung 1 angepaßt ist und der in dieser Öffnung mit leichtem Haftsitz eindrückbar ist, wobei der Verschlußdeckel 2 wieder aus dieser Einfüllöffnung herausgezogen werden kann.

Um einen glatten Abschluß und einen gewissen Schutz gegen ein unbefugtes Öffnen zu erzielen, kann die Einfüllöffnung an ihrem oberen Ende erweitert sein, so daß sie einen Rand 6 des Verschlußdeckels 2 aufnehmen kann.

Der Verschlußdeckel 2 weist weiterhin eine Lasche 4 auf, die mit dem Verschlußdeckel 2 über ein Filmscharnier verbunden ist, so daß der außenliegende Teil der Lasche 4 gegenüber dem Behälterdeckel in Richtung des Flansches 5 senkrecht abklappbar ist.

Der Abfallbehälterdeckel 10 ist mit einer Verschlußhülse 7 versehen, die sich in vorteilhafter Weise am Außenumfang des Abfallbehälterdeckels 10 befindet, jedoch auch in einem Innenbereich des Abfallbehälterdeckels und damit nach dessen Aufsetzen auf den Abfallbehälterkörper im Inneren dieses Abfallbehälterkörpers liegen kann.

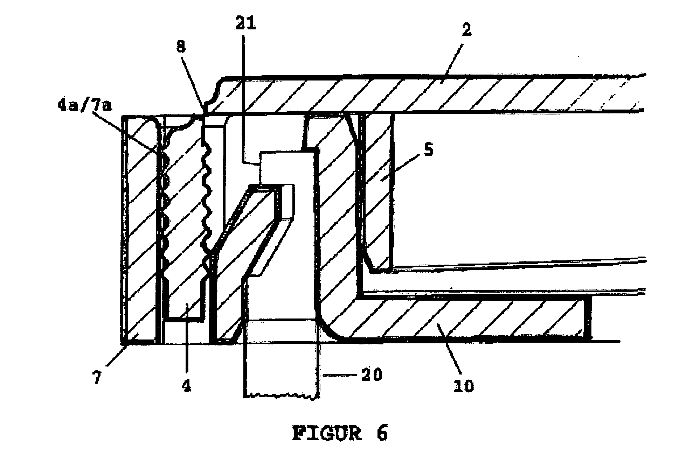

Wenn das freie Ende der Lasche 4 um das Filmscharnier 8 herum senkrecht abgewinkelt ist, so kann dieses freie Ende in die Verschlußhülse 7 eingeführt werden, und im Inneren der Verschlußhülse 7 angeordnete Rasteinrichtungen 7a wirken dann mit auf der Lasche ausgebildeten Rasteinrichtungen 4a zusammen, um diese Lasche sicher und unlösbar in der Verschlußhülse 7 zu verriegeln, so daß ein Öffnen des Verschlußdeckels in dieser Schließstellung nur noch mit extremer Gewalt möglich ist. Dies ist beispielsweise aus Figur 6 zu erkennen, bei der das freie Ende der Lasche 4 rechtwinklig zum Behälterdeckel 2 abgeklappt ist und im Inneren der Verschlußhülse 7 liegt, wobei die Rastverzahnungen 4a, 7a fest miteinander in Eingriff stehen. Die Anzahl der Zähne der Rastverzahnungen ist hier lediglich als ein Beispiel dargestellt, und es ist beispielsweise möglich, eine Vielzahl von Rastverzahnungen auf dem freien Teil der Lasche 4 anzuordnen, während im Inneren der Verschlußhülse lediglich eine oder zwei Rastzähne angeordnet sind. Eine verbesserte Verrastung ergibt sich jedoch mit einer größeren Anzahl von Zähnen der Rastverzahnung. Die Lasche 4 kann in der in Figur 6 ersichtlichen Weise mit relativ engem Sitz im Inneren der Verschlußhülse 7 liegen, so daß es selbst unter Verwendung von Werkzeugen schwierig ist, die Rastverzahnungen voneinander zu trennen. Weiterhin kann die Verschlußhülse 7 gemäß Figur 6 an ihrem unteren Ende verschlossen sein, so daß der Einsatz eines Werkzeuges an dieser Stelle nicht möglich ist.

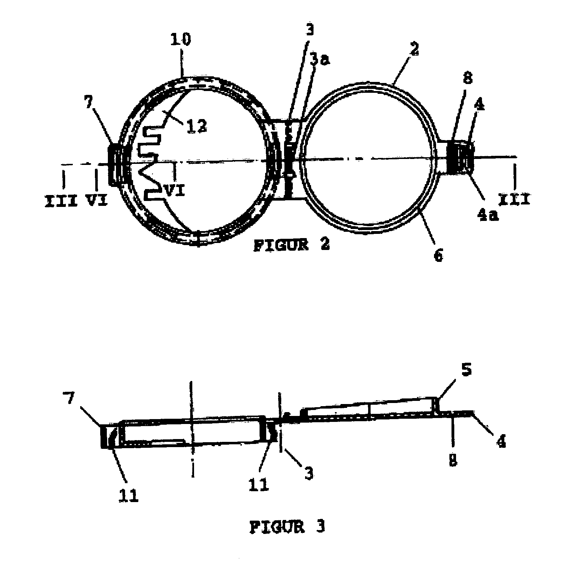

Wie dies aus den Figuren 1-3 und 6 zu erkennen ist, weist der Abfallbehälterdeckel 10 weiterhin am Umfang verteilte Rastlaschen 11 auf, die unter einen Wulst 21 gemäß Figur 6 am oberen Ende eines Abfallbehälterkörpers 20 greifen und damit den Abfallbehälterdeckel unlösbar mit dem Abfallbehälterkörper 20 verbinden.

Wie dies weiterhin in den Figuren 1-3 gezeigt ist, kann im Bereich des den Verschlußdeckel 2 mit dem Abfallbehälterdeckel 10 verbindenden Filmscharniers 3 ein Rastelement angeordnet sein, das unter leichter Rastwirkung den Verschlußdeckel in geöffneten Zustand halten kann.

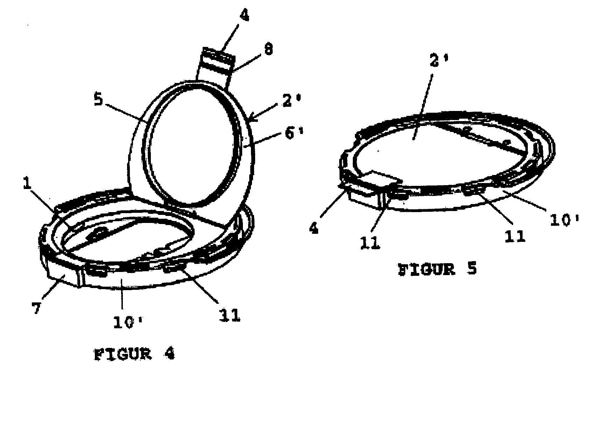

In den Figuren 4 und 5 ist eine weitere Ausführungsform eines Abfallbehälterdeckels 10' mit einem Verschlußdeckel 2' dargestellt, der sich von dem Verschlußdeckel 2 nach den in Figuren 1-3 dadurch unterscheidet, daß der Rand 6' wesentlich verbreitert ist und eine relativ kleine Einfüllöffnung 1 abdeckt. Aus Figur 5 ist weiterhin die erste Verschlußstellung zu erkennen, in der der Verschlußdeckel 2' über seinen Flansch 5 in die Einfüllöffnung 1 eingesetzt ist und mit leichtem Haftsitz in dieser hält. Die Lasche 4 erstreckt sich in Verlängerung der Hauptebene des Verschlußdeckels 2' über den Außenumfang des Abfallbehälterdeckels 10' hinaus, so daß sie als Öffnungshilfe zum Aufziehen des Verschlußdeckels 2' dienen kann.

Gleiches gilt selbstverständlich für die in den Figuren 1-3 dargestellte Ausführungsform.
Obwohl bei der vorstehenden Beschreibung ein getrennter Abfallbehälterdeckel 10 bzw. 10' verwendet wird, der auf den oberen Rand eines Abfallbehälterkörpers 20 gemäß Figur 6 aufgesetzt ist, ist es selbstverständlich in gleicher Weise möglich, den Verschlußdeckel 2 über ein Filmschamier direkt an dem oberen Ende des Abfallbehälters zu befestigen, der dann an einer dem Filmschamier gegenüberliegenden Position die Verschlußhülse aufweist.

Dies kann insbesondere bei sehr kleinen Abfallbehältern sinnvoll sein, bei denen ein zusätzlicher Abfallbehälterdeckel nicht erforderlich ist.

Obwohl in den beschriebenen Ausführungsformen die Lasche 4 und die Verschlußhülse 7 mit einem rechtwinkligen Querschnitt dargestellt sind, ist es in gleicher Weise möglich, beispielsweise die Lasche mit einem runden Querschnitt auszugestalten, der eine Außenverzahnung aufweist, die in einen mit Innenverzahnung ausgebildete Verschlußhülse mit rundem Innenquerschnitt paßt.

## Patentansprüche

1. Abfallbehälter, insbesondere für medizinische Abfälle, mit einer Einfüllöffnung (1), die durch einen Verschlußdeckel (2) verschließbar ist, der mit dem Abfallbehälter über eine Gelenkverbindung (3) gelenkig verbunden ist, wobei zur Arretierung des Verschlußdeckels (2) nach dem Befüllen des Abfallbehälters bezüglich der Einfüllöffnung (1) Rasteinrichtungen (4, 7) vorgesehen sind.
**dadurch gekennzeichnet, daß** der Verschlußdeckel (2) an einer der Gelenkverbindung (3) gegenüberliegenden Position eine sich nach außen hin erstreckende Lasche (4) aufweist, die gelenkig mit dem Verschlußdeckel (2) verbunden ist und einen ersten Teil der Rasteinrichtungen bildet, daß der Abfallbehälter eine Verschlußhülse (7) aufweist, in die die Lasche (4) einsteckbar ist und die einen zweiten Teil der Rasteinrichtungen bildet, und daß auf der Außenseite der Lasche (4) und der Innenseite der Verschlußhülse (7) jeweils zusammenwirkende Rastverzahnungen (4a, 7a) angeordnet sind, die einen unlösbaren Rasteingriff zwischen der Lasche (4) und der Verschlußhülse (7) beim Einstecken der Lasche (4) in die Verschlußhülse (7) ergeben.

2. Abfallbehälter nach Anspruch 1,
**dadurch gekennzeichnet, daß** der Verschlußdeckel (2) über weitere lösbare Rasteinrichtungen (5) bezüglich der Einfüllöffnung (1) in einer ersten Stellung arretierbar ist, in der der Verschlußdeckel (2) mit Hilfe der Lasche wieder geöffnet werden kann.

3. Abfallbehälter nach Anspruch 1 oder 2,
**dadurch gekennzeichnet, daß** der Verschlußdeckel (2) aus Kunststoff besteht, und daß die Lasche (4) über ein Filmschamier (8) mit dem Verschlußdeckel (2) verbunden ist.

4. Abfallbehälter nach einem der vorhergehenden Ansprüche,
**dadurch gekennzeichnet, daß** sich die Lasche (4) im Ruhezustand in wesentlichen parallel zur Hauptebene des Verschlußdeckels (2) erstreckt und zum Einstecken in die Hülse (7) um die Gelenkverbindung (8) abwinkelbar ist.

5. Abfallbehälter nach einem der vorhergehenden Ansprüche,
**dadurch gekennzeichnet, daß** das Innenvolumen des Abfallbehälters im wesentlichen durch einen Abfallbehälterkörper gebildet ist, und daß ein Abfallbehälterdeckel (10) vorgesehen ist, in dem die Einfüllöffnung (1) angeordnet ist.

6. Abfallbehälter nach Anspruch 5,
**dadurch gekennzeichnet, daß** der Verschlußdeckel (2) gelenkig an dem Abfallbehälterdeckel (10) befestigt ist.

7. Abfallbehälter nach Anspruch 5 oder 6,
**dadurch gekennzeichnet, daß** die Verschlußhülse (7) an dem Abfallbehälterdeckel (10) befestigt ist.

8. Abfallbehälter nach einem der Ansprüche 5 bis 7,
**dadurch gekennzeichnet, daß** der Abfallbehälterdeckel (10) unlösbar und flüssigkeitsdicht mit dem Behälterkörper über dritte Rasteinrichtungen (11) verbindbar ist.
